# EUROPÄISCHE PATENTSCHRIFT

(11) **EP 0 980 362 B1**
(45) Veröffentlichungstag und Bekanntmachung des Hinweises auf die Patenterteilung: **16.08.2006**
(21) Anmeldenummer: 98933446.1
(22) Anmeldetag: 30.04.1998
(51) Int. Cl.: C07D 265/36, C07D 265/34, C07D 279/16, A61K 31/535

(54) **SUBSTITUIERTE HETEROCYCLEN UND DEREN VERWENDUNG IN ARZNEIMITTELN**
SUBSTITUTED HETEROCYCLES AND THEIR USE IN MEDICAMENTS
HETEROCYCLIQUES SUBSTITUES ET LEUR UTILISATION DANS DES MEDICAMENTS

(30) Priorität: 02.05.1997 DE 19720155
(43) Veröffentlichungstag der Anmeldung: 23.02.2000
(73) Patentinhaber: Schering Aktiengesellschaft, 13353 Berlin (DE)
(72) Erfinder: HÖLSCHER, Peter, D-10559 Berlin (DE); REHWINKEL, Hartmut, D-12051 Berlin (DE); SUELZLE, Detlev, D-12163 Berlin (DE); BURTON, Gerardine, D-13591 Berlin (DE); HILLMANN, Margrit, D-13437 Berlin (DE); PRIBILLA, Iris, D-14055 Berlin (DE); DAVEY, David, Daniel, El Sobrante, CA 94803 (US)
(74) Vertreter: Pohlman, Sandra M.
(86) Internationale Anmeldenummer: PCT/DE1998/001241
(87) Internationale Veröffentlichungsnummer: WO 1998/050372

(56) Entgegenhaltungen:
- WO-A-95/05363
- WO-A-96/14844
- CHEMICAL ABSTRACTS, vol. 127, no. 3, 21. Juli 1997 Columbus, Ohio, US; abstract no. 34228, MIYAKOSHI T. ET AL.: "Preparation of 1,4-benzothiazine derivatives by cyclization" XP002077199 & JP 09 124626 A (SOGO YATSUKO K.K.) 13. Mai 1997

## Beschreibung

Die Erfindung betrifft substituierte Heterocyclen, das Verfahren zu deren Herstellung und deren Verwendung in Arzneimitteln.

In menschlichen Zellen existieren 3 spezifische Formen von Stickstoffmonoxid-Synthasen, die Arginin in Stickstoffmonoxid (NO) und Citrullin überführen. So wurden zwei konstitutive NO-Synthasen (NOS) identifiziert, die als Ca⁺⁺/Calmodulin abhängige Enzyme im Gehirn (bcNOS oder NOS 1) bzw. im Endothel (ecNOS oder NOS 3) vorhanden sind. Die dritte Isoform ist die induzierbare NOS (iNOS oder NOS 2), die ein Ca⁺⁺ unabhängiges Enzym ist und induziert wird nach Aktivierung unterschiedlicher Zellen durch Endotoxin und Cytokine.

NOS-Inhibitoren und insbesondere spezifische Inhibitoren der NOS 1, NOS 2 oder NOS 3 sind daher zur Therapie unterschiedlicher Erkrankungen geeignet, die durch pathologische Konzentrationen von NO in Zellen hervorgerufen oder verschlimmert werden (Clin. Neuropharmac. 18, 1995 Seite 482).

Als NOS-Inhibitoren werden unterschiedliche Verbindungen beschrieben wie beispielsweise cyclische Amidinderivate (WO 96/14844) oder Guanidinderivate (WO95/05363).

Es wurde nun gefunden, daß die erfindungsgemäß substituierten Heterocyclen besonders vorteilhaft als Arzneimittel eingesetzt werden können.

Die Erfindung betrifft die Verbindungen der Formel I, deren tautomere und isomere Formen und Salze 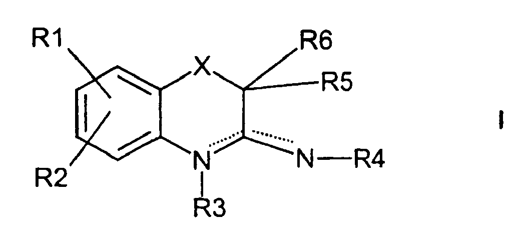 worin 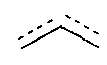 eine Doppelbindung,
X -O- oder -S(O)ₘ-,

R¹ und R² unabhängig voneinander Wasserstoff, Halogen, S(O)ₙ-R⁷, OR⁷, COOR⁷, NR⁷R⁸, C(=NR⁷)-NHR⁸, C₁₋₆-Alkyl, C₂₋₆-Alkenyl, C₂₋₆-Alkynyl oder -S-C(=NR⁷)-NHR⁸,

R³ und R⁴ unabhängig voneinander Wasserstoff, C₁₋₁₂-Alkyl, Phenyl, CO-NR⁹R¹⁰, CSNR⁹R¹⁰, COR⁹, CSR⁹, COOR⁹, OH, O-C₁₋₆-Alkyl,
und

R⁵ Halogen, C₁₋₈-Alkoxy, S(O)ₚ-C₁₋₆-Alkyl, C₁₋₈-Alkylcarbonyl, C₁₋₈-Alkyl, C₃₋₁₀₋Cycloalkyl, Phenyl oder einen C₁₋₈-Alkylrest, der mit Phenyl, Halogen, Hydroxy, S(O)ₙR¹¹, NO₂, OR¹¹, COOR¹¹, NR¹¹R¹², Cyano, -C(=NR¹¹)-NHR¹² oder -S-C(=NR¹¹)-NHR¹² substituiert ist.

R⁶ Wasserstoff oder C₁₋₃-Alkyl, das gegebenenfalls gemeinsam mit R⁵ einen 3-, 4-oder 5-gliedrigen Spirocyclus bildet,

R⁷, R⁸ und R¹¹, R¹² gleich oder verschieden Wasserstoff, C₁₋₆-Alkyl, Phenyl oder C₃₋₇-Cycloalkyl,

R⁹ und R¹⁰ unabhängig voneinander Wasserstoff, Phenyl, Benzyl, C₃₋₇-Cycloalkyl oder C₁₋₆-Alkyl,

m, n, p 0, 1 oder 2 ist.

Als eine bevorzugte Ausführungsform sind die Verbindungen der Formel I zu betrachten, worin R³ oder R⁴ Wasserstoff bedeutet. Eine weitere bevorzugte Ausführungsform sind Verbindungen, in denen R⁵ C₁₋₈-Alkyl ist. R⁶ ist bevorzugt Wasserstoff. Besonders bevorzugt sind Verbindungen, in denen R³, R⁴ und R⁶ Wasserstoff bedeuten. Die Definition von m, n und p ist vorzugsweise null.

Die Verbindungen der Formel I können als Tautomere, Stereoisomere oder geometrische Isomere vorliegen. Die Erfindung umfaßt auch alle möglichen Isomeren wie E- und Z-Isomere, S- und R-Enantiomere, Diastereomere, Razemate und Gemische derselben einschließlich der tautomeren Verbindungen der Formel la und Ib

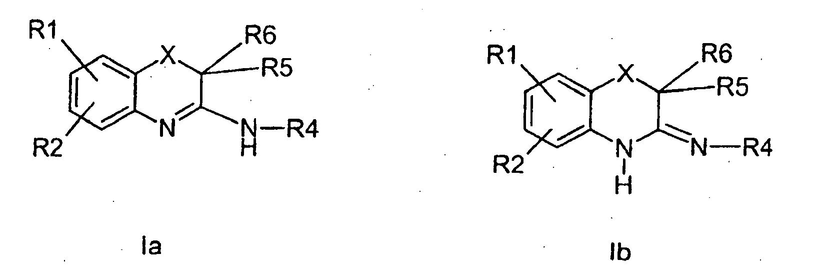

Die physiologisch verträglichen Salze können mit anorganischen und organischen Säuren gebildet werden wie beispielsweise Oxalsäure, Milchsäure, Zitronensäure, Fumarsäure, Essigsäure, Maleinsäure, Weinsäure, Phosphorsäure, HCl, HBr, Schwefelsäure, p-Toluolsulfonsäure, Methansulfonsäure u.a.

Zur Salzbildung von Säuregruppen sind auch die anorganischen oder organischen Basen geeignet, die zur Bildung physiologisch verträglicher Salze bekannt sind wie beispielsweise Alkalihydroxide, wie Natrium- und Kaliumhydroxid, Erdalkalihydroxide wie Calciumhydroxid, Ammoniak, Amine wie Ethanolamin, Diethanolamin, Triethanolamin, N-Methylglucamin, Tris-(hydroxymethyl)-methylamin usw.

Alkyl bedeutet jeweils eine geradkettige oder verzweigte Alkylgruppe wie z.B. Methyl, Ethyl, Propyl, Isopropyl, n-Butyl, sek. Butyl, tert. Butyl, n-Pentyl, sek. Pentyl, tert. Pentyl, Neopentyl, n-Hexyl., sek. Hexyl, Heptyl, Octyl, vorzugsweise Alkylreste mit 1- 4 C-Atomen. Die Alkenyl- und Alkynyl-Substituenten sind jeweils geradkettig oder verzweigt. Beispielsweise seien die folgenden Reste genannt: Vinyl, 2-Propenyl, 1-Propenyl, 1-Butenyl, 2-Butenyl, 1-Methyl-1-propenyl, 2-Methyl-2-propenyl, 3-Methyl-2-propenyl, Ethinyl, 1-Propinyl, 2-Propinyl, 1-Butinyl, 2-Butinyl.

Unter Cycloalkyl ist jeweils Cyclopropyl, Cyclobutyl, Cyclopentyl, Cyclohexyl oder Cycloheptyl zu verstehen.

Halogen bedeutet jeweils Fluor, Chlor, Brom oder Jod.

Unter Aryl ist Naphthyl oder Phenyl zu verstehen.

Beispielsweise seien die folgenden Spirocyclen genannt:
Spiropropyl, Spirobutyl, Spiropentyl.

Der Alkylrest R⁵ kann ein- bis mehrfach mit den genannten Substituenten substituiert sein.

Die Erfindung betrifft auch die Verwendung der erfindungsgemäßen Verbindungen zur Herstellung eines Arzneimittels zur Behandlung von Erkrankungen, die durch die Wirkung von Stickstoffmonoxid in pathologischen Konzentrationen hervorgerufen werden. Dazu zählen neurodegenerative Erkrankungen, inflammatorische Erkrankungen, Autoimmunerkrankungen, Herz-Kreislauf-Erkrankungen. Beispielsweise seien genannt:
Cerebrale Ischaemie, Hypoxie und andere neurodegenerative Erkrankungen, die mit Entzündungen in Verbindung gebracht werden wie Multiple Sklerose, Amyotrophe Lateralsklerose und vergleichbare sklerotische Erkrankungen, Morbus Parkinson, Huntington's Disease, Korksakoff's Disease, Epilepsie, Schlafstörungen, Schizophrenie, Depression, Migräne, Hypoglykämie, Demenz wie z.B. Alzheimersche Krankheit, HIV-Demenz und Presenile Demenz.
Ferner eignen sie sich zur Behandlung von Krankheiten des Herz-Kreislauf Systems und zur Behandlung autoimmuner und/oder inflammatorischer Erkrankungen wie Hypotension, ARDS (adult respiratory distress syndrome), Sepsis oder Septischer Schock, Rheumatoider Arthritis, Osteoarthritis, von insulinabhängiger Diabetes Mellitus (IDDM), entzündlicher Erkrankung des Beckens /Darms (bowel disease), von Meningitis, Glomerulonephritis, akute und chronische Lebererkrankungen, Erkrankungen durch Abstoßung (beispielsweise allogene Herz-,Nieren- oder Lebertransplantationen) oder entzündlichen Hautkrankheiten wie Psoriasis und andere. Auf Grund ihres Wirkprofils eignen sich die erfindungsgemäßen Verbindungen sehr gut zur spezifischen Inhibition der neuronalen NOS und können daher zur Behandlung von neurodegenerativer Erkrankungen wie Stroke eingesetzt werden.
Zur Verwendung der erfindungsgemäßen Verbindungen als Arzneimittel werden diese in die Form eines pharmazeutischen Präparats gebracht, das neben dem Wirkstoff für die enterale oder parenterale Applikation geeignete Träger-, Hilfs- und/oder Zusatzstoffe enthält. Die Applikation kann oral oder sublingual als Feststoff in Form von Kapseln oder Tabletten oder als Flüssigkeit in Form von Lösungen, Suspensionen, Elixieren, Aerosolen oder Emulsionen oder rektal in Form von Suppositorien oder in Form von gegebenenfalls auch subcutan intramuskulär oder intravenös anwendbaren Injektionslösungen oder topisch oder intrathekal erfolgen. Als Hilfsstoffe für die gewünschte Arzneimittelformulierung sind die dem Fachmann bekannten inerten organischen und anorganischen Trägermaterialien geeignet wie z. B. Wasser, Gelatine, Gummmi arabicum, Milchzucker, Stärke, Magnesiumstearat, Talk, pflanzliche Öle, Polyalkylenglykole usw. Gegebenenfalls können darüber hinaus Konservierungs-, Stabilisierungs-, Netzmittel, Emulgatoren oder Salze zur Veränderung des osmotischen Druckes oder Puffer enthalten sein.
Für die parenterale Anwendung sind insbesondere Injektionslösungen oder Suspensionen, insbesondere wäßrige Lösungen der aktiven Verbindungen in polyhydroxyethoxyliertem Rizinusöl, geeignet.
Als Trägersysteme können auch grenzflächenaktive Hilfsstoffe wie Salze der Gallensäuren oder tierische oder pflanzliche Phospholipide, aber auch Mischungen davon sowie Liposome oder deren Bestandteile verwendet werden.
Für die orale Anwendung sind insbesondere Tabletten, Dragees oder Kapseln mit Talkum und/oder Kohlenwasserstoffträger oder -binder, wie zum Beispiel Lactose, Mais- oder Kartoffelstärke, geeignet. Die Anwendung kann auch in flüssiger Form erfolgen, wie zum Beispiel als Saft, dem gegebenenfalls ein Süßstoff beigefügt ist.

Die Dosierung der Wirkstoffe kann je nach Verabfolgungsweg, Alter und Gewicht des Patienten, Art und Schwere der zu behandelnden Erkrankung und ähnlichen Faktoren variieren. Die tägliche Dosis beträgt 1-2000 mg, vorzugsweise 20-500 mg, wobei die Dosis als einmal zu verabreichende Einzeldosis oder unterteilt in 2 oder mehreren Tagesdosen gegeben werden kann.

Die NOS-inhibitorische Wirksamkeit der Verbindungen der Formel 1 und deren physiologisch verträglicher Salze kann in Anlehnung an die Methoden von Bredt und Snyder in Proc. Natl. Acad. Sci. USA (1989) 86, 9030 mit nachfolgend genannten Varianten bestimmt werden.

Die Wirkung der genannten Verbindungen wird durch folgende Beispiele illustriert.

| Substanz | IC₅₀ bcNOS in µM | IC₅₀ ecNOS in µM | IC₅₀ iNOS in µM |
|---|---|---|---|
| 2,8-Dimethyl-3-amino-1,4-benzoxazin | 20 | >100 | >10 |
| 2,6,7-Trimethyl-3-amino-1,4-benzoxazin | 3 | >10 | >100 |
| 2-n-Propyl-3-amino-1,4-benzoxazin | 1,2 | >10 | >100 |

Inhibitionsassay mit recombinanter humaner brain NO-Synthase (bcNOS). (Konzentration: 0.022 IU/I). Gemessener Parameter: % Inhibition. durch Testverbindung. Substrat: [3H]-Arginin (Konzentration 4.4E-08 mol/l ). Analog mit recombinanter humaner endothel NO-Synthase (ecNOS) und recombinanter humaner induzierbarer NO-Synthase (iNOS).

Die Herstellung der erfindungsgemäßen Verbindungen erfolgt dadurch; daß man
a) eine Verbindung der Formel II oder deren Salz 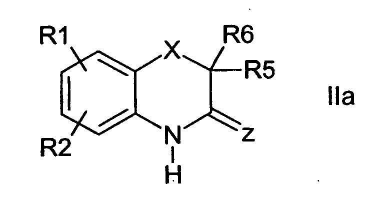 oder 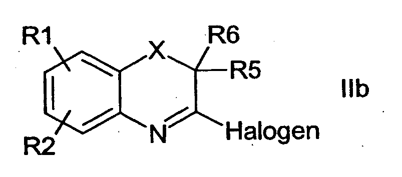 worin
   R¹, R² und X die obige Bedeutung haben und Z Sauerstoff oder Schwefel ist, mit Ammoniak oder primären oder sekundären Aminen umsetzt oder
b) eine Verbindung der Formel III 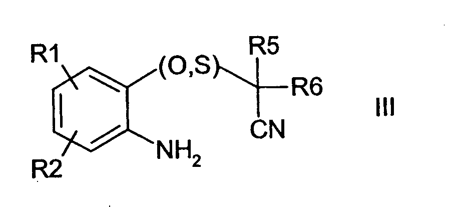 worin

R¹, R², R⁵, R⁶ die obige Bedeutung haben, cyclisiert und gewünschtenfalls anschließend Sulfide oxidiert, Ester verseift, Säuren verestert, Hydroxygruppen verethert oder acyliert, Amine acyliert, alkyliert oder diazotiert, halogeniert, NO₂ einführt oder reduziert, mit Thioharnstoffderivaten umsetzt, die Isomeren trennt oder die Salze bildet.

Die Umsetzung nach Verfahrensvariante a) mit Ammoniak gelingt unter Druck in Autoklaven bei Ammoniaküberschuß bei tiefen Temperaturen (- 78 °C) oder durch Rühren in mit Ammoniak gesättigtem Methanol. Wird mit Aminen umgesetzt, so stellt man aus dem Lactam oder Thiolactam zunächst den Iminether oder Iminothioether als Zwischenverbindung dar (z.B. mit Methyliodid oder Methylsulfat) und setzt diesen mit oder ohne Isolierung der Zwischenverbindung mit den entsprechenden Aminen oder deren Salzen um.

Alternativ können nach der Verfahrensvariante b) die erfindungsgemäßen Verbindungen aus Aminonitrilen erhalten werden, indem man mit einer Base wie z.B. Alkalialkoholat cyclisiert.

Die sich gegebenenfalls anschließende Verseifung einer Estergruppe kann basisch oder sauer erfolgen, indem man bei Raumtemperatur oder erhöhter Temperatur bis zur Siedetemperatur des Reaktionsgemisches in Gegenwart von Alkalihydroxiden in Ethanol oder anderen Alkoholen oder mittels Säuren wie z.B. Salzsäure hydrolysiert und gegebenenfalls Salze der Aminobenzoxazine oder -thiazine weiterverarbeitet.

Die Veresterung der Carbonsäure geschieht in an sich bekannter Weise mit Diazomethan oder dem entsprechenden Alkohol in Säure oder in Gegenwart eines aktivierten Säurederivats. Als aktivierte Säurederivate kommen zum Beispiel Säurechlorid, -imidazolid oder -anhydrid in Frage.

Die Synthese von Sulfoxiden und Sulfonen gelingt beispielsweise mit Persäuren wie m-Chlorperbenzoesäure in bekannter Weise. Halogenierte Seitenketten können mit Thioharnstoffen in üblicher Weise zu lsothioharnstoffderivaten umgesetzt werden.

Zusätzlich kann durch elektrophile aromatische Substitution eine Nitrogruppe oder Halogen, insbesondere Brom, eingeführt werden. Dabei entstehende Gemische können in üblicher Weise, auch mittels HPLC, getrennt werden. Verbindungen, die mit Amidin substituiert sind, werden aus dem Nitril durch Addition von Aminoverbindungen hergestellt.

Die Reduktion der Nitrogruppe oder gegebenfalls der Cyanogruppe zur Aminogruppe erfolgt katalytisch in polaren Lösungsmitteln bei Raumtemperatur oder erhöhter Temperatur unter Wasserstoffdruck. Als Katalysatoren sind Metalle wie Raney-Nickel oder Edelmetallkatalysatoren wie Palladium oder Platin gegebenenfalls in Gegenwart von Bariumsulfat oder auf Trägern geeignet. Statt Wasserstoff kann auch Ammoniumformiat in bekannter Weise benutzt werden. Reduktionsmittel wie Zinn-II-chlorid oder Titan-III-chlorid können ebenso verwendet werden wie komplexe Metallhydride eventuell in Gegenwart von Schwermetallsalzen. Es kann vorteilhaft sein vor der Reduktion die Estergruppe einzuführen. Für Nitrogruppen bewährt hat sich die Reduktion mit Zink in Essigsäure.

Wird eine Alkylierung einer Aminogruppe gewünscht, so kann nach üblichen Methoden beispielsweise mit Alkylhalogeniden alkyliert werden.

Die Acylierung der Aminogruppe erfolgt in üblicher Weise beispielsweise mit einem Säurehalogenid oder Säureanhydrid gegebenenfalls in Gegenwart einer Base.

Die Einführung der Halogene Chlor, Brom oder Jod über die Aminogruppe kann beispielsweise auch nach Sandmeyer erfolgen, indem man die mit Nitriten intermediär gebildete Diazoniumsalze mit Cu(I)chlorid oder Cu(I)bromid in Gegenwart der entsprechenden Säure wie Salzsäure oder Bromwasserstoffsäure umsetzt oder mit Kaliumjodid umsetzt.

Die Einführung einer NO₂-Gruppe gelingt durch eine Reihe von bekannten Nitrierungsmethoden. Beispielsweise kann mit Nitroniumtetrafluoroborat in inerten Lösungsmitteln wie halogenierten Kohlenwasserstoffen oder in Sulfolan oder Eisessig nitriert werden. Möglich ist auch die Einführung z.B. durch Nitriersäure in Wasser oder konz. Schwefelsäure als Lösungsmittel bei Temperaturen zwischen 0°C und 30°C.

Hydroxygruppen werden in bekannter Weise verestert oder verethert.

Die Isomerengemische können nach üblichen Methoden wie beispielsweise Kristallisation, Chromatographie oder Salzbildung in die Enantiomeren bzw. E/Z-Isomeren aufgetrennt werden. Die Enantiomeren können auch durch stereoselektive Synthese oder durch Auftrennung nach einzelnen Reaktionsschritten erhalten werden.

Die Herstellung der Salze erfolgt in üblicher Weise, indem man eine Lösung der Verbindung der Formel I mit der äquivalenten Menge oder einem Überschuß einer Säure, die gegebenenfalls in Lösung ist, versetzt und den Niederschlag abtrennt oder in üblicher Weise die Lösung aufarbeitet.

Soweit die Herstellung der Ausgangsverbindungen nicht beschrieben wird, sind diese bekannt und käuflich oder analog zu bekannten Verbindungen oder nach hier beschriebenen Verfahren herstellbar.

Die Herstellung der Lactame der Formel II kann beispielsweise dadurch erfolgen, daß man eine Verbindung der Formel IV 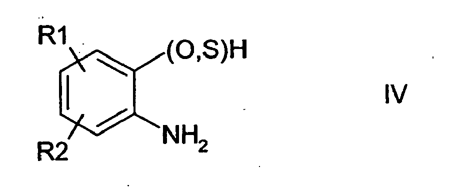 worin R¹ und R² die obige Bedeutung haben mit einer Verbindung der Formel V 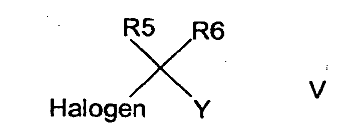 worin R⁵ und R⁶ die obige Bedeutung haben und Y eine reaktive Carboxylgruppe ist wie Säurehalogenid, Nitril oder Carbonsäureester umsetzt und cyclisiert oder dadurch, daß man eine Verbindung der Formel VI 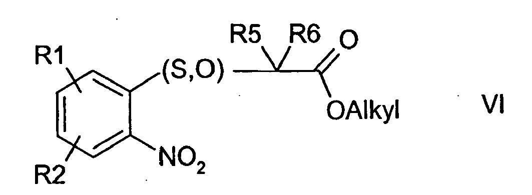 nach Reduktion cyclisiert.

Neue Verbindungen wurden durch eine oder mehrere der folgenden Methoden charakterisiert: Schmelzpunkt, Massenspektroskopie, Infrarotspektroskopie, Nuklearmagnetische Resonanzspektroskopie (NMR). NMR Spektren wurden mit einem Bruker 300 MHz Gerät gemessen, die (deuterierten) Lösemittel werden jeweils angegeben und wie folgt abgekürzt: CDCl₃ (Chloroform), DMSO (Dimethylsulfoxid). Verschiebungen sind in delta und ppm angegeben. Femer bedeuten: THF (Tetrahydrofuran), DMF (N,N-Dimethylformamid), MeOH (Methanol), EE (Ethylacetat). Alle Lösemittel sind p.A.Qualität, wenn nicht anders vermerkt. Alle Reaktionen werden unter Schutzgas ausgeführt, es sei denn es handelt sich um wässrige Lösungen. Es bedeuten:
mult. (Multiplett, mehrere Signale), s (Singulett), d (Dublett), dd (Doppeldublett usw.), tr (Triplett), H (Wasserstoffprotonen), J (Kopplungskonstante), ml (Milliliter), RT (Raumtemperatur). Schmelzpunkte werden in Grad Celsius angegeben und sind nicht korrigiert. Die Ausbeuten in Prozent beziehen sich auf das Edukt, nicht auf den Umsatz.

Nachfolgend wird die Darstellung einiger Vorstufen, Zwischenprodukte und Produkte exemplarisch beschrieben.

### Ausgangsverbindungen

### A) 2-Phenyl-2H-1,4-benzoxazin-3-on

2.18 g 2-Aminophenol werden in 13 ml Methylethylketon mit 4 g Natriumhydrogencarbonat in 13 ml Wasser versetzt und bei Eisbadtemperatur tropfenweise mit 3,63 ml D,L-2-Chlor-2-phenylacetylchlorid versetzt. Nach erwärmen auf 70 °C wird auf Eiswasser gegossen, mit Essigester extrahiert, die organische Phase mit Magnesiumsulfat getrocknet und eingeengt. Es resultieren 6,3 g Rohprodukt, das aus Isopropylether umkristallisiert wird.
Schmelzpunkt 146 °C.

### B) 2,2-Dimethyl-1,4-benzoxazin-3-on

3 g 2-Aminophenol werden in 23 ml Toluol mit 1,8 g Natriumhydrid unter Zusatz von 50 mg TEBA (Triethylbenzylammoniumchlorid) versetzt und bei Eisbadtemperatur tropfenweise 4,4 ml 2-Brom-2-methylpropionsäureethylester zugegeben. Nach 2 Stunden bei Raumtemperatur wird auf Eiswasser gegossen, mit Essigester extrahiert, mit Sole gewaschen, die organische Phase mit Magnesiumsulfat getrocknet und eingeengt. Es resultieren nach Umkristallisieren aus Isopropylether 278 mg Kristalle.
[1H]-NMR (DMSO): 10,5 1H, 6,9 m 4H, 1,4 s 6H.

Auf die gleiche Weise werden hergestellt:
2-Methyl-1,4-benzoxazin-3-on
aus D,L- 2-Brompropionsäureethylester.
2-(2-Phenylethyl)-1,4-benzoxazin-3-on
[1H]-NMR (DMSO): 10,6 1H breit, 7,3-7,2 m 5H, 4,49 dd 1H, 2,8 m 2H, 2,1 m 2H.
2-Benzyl-1,4-benzoxazin-3-on
2-Ethyl-1,4-benzoxazin-3-on
[1H]-NMR (CDCl₃): 8,7 breit 1 H, 7,0 bis 6,8 m 4H, 4,52 dd 1H, 1,98 m 2H, 1,11 tr 3H.
2-Propyl-1,4-benzoxazin-3-on
[1H]-NMR (CDCl₃): 8,65 breit 1H, 7,0 bis 6,8 m 4H, 4,59 dd 1H, 1,89 m 2H, 1,6 m 2H, 1,00 tr
3H.
2-(2-Propyl)-1,4-benzoxazin-3-on
2-tert. Butyl-1,4-benzoxazin-3-on
2-n-Butyl-1,4-benzoxazin-3-on
2-n-Hexyl-1,4-benzoxazin-3-on
2-Carboxymethyl-1,4-benzoxazin-3-on
2,2-*spiro*-Butyl-1,4-benzoxazin-3-on
2-Acetyl-1,4-benzoxazin-3-on

### C) 2,6-Dimethyl-1,4-benzoxazin-3-on

3,39 g 2-Amino-4-methylphenol werden in 12 ml Toluol mit 1,8 g Natriumhydrid versetzt und bei Eisbadtemperatur tropfenweise 3,95 ml 2-Brom-propionsäureethylester zugegeben. Nach 4 Stunden bei Raumtemperatur wird auf Wasser gegossen, mit mit Essigester extrahiert, mit Sole gewaschen, die organische Phase mit Magnesiumsulfat getrocknet und eingeengt. Säulenchromatografie mit Hexan/Essigester liefert 43% Produkt.

Auf die gleiche Weise werden hergestellt:
2, 5-Dimethyl-1,4-benzoxazin-3-on
2,7-Dimethyl-1,4-benzoxazin-3-on
2,8-Dimethyl-1,4-benzoxazin-3-on
2-Methyl-6-chlor-1,4-benzoxazin-3-on
aus 2-Amino-4-chlorphenol
2-Methyl-7-methylcarboxy-1,4-benzoxazin-3-on
2-Methyl-6,8-dichlor-1,4-benzoxazin-3-on
2-Methyl-6-*tert-*butyl-1,4-benzoxazin-3-on
2,6,7-Trimethyl-1,4-benzoxazin-3-on
2,6,8-Trimethyl-1,4-benzoxazin-3-on

### D) 2-Methyl-1,4-benzothiazin-3-on

2,95 g 2-Amino-thiophenol werden in 6 ml Toluol mit 1,8 g Natriumhydrid versetzt und bei Eisbadtemperatur werden tropfenweise 3,95 ml 2-Brom-propionsäureethylester zugegeben. Nach 2 Stunden bei Raumtemperatur wird auf Wasser gegossen, mit mit Essigester extrahiert, mit Sole gewaschen, die organische Phase mit Magnesiumsulfat getrocknet und eingeengt.
Säulenchromatografie mit Hexan/Essigester liefert das Produkt. [1H]-NMR (CDCl₃):8,83 s breit 1H, 7,32 dd 1H, 7,19 dtr 1H, 7,03 dtr 1H, 6,90 dd 1H, 3,57 q 1H, 1,51 d 3H.

Auf die gleiche Weise werden hergestellt:
2-Ethyl-2H-1,4-benzothiazin-3-on
2-(2-Propyl)-2H-1,4-benzothiazin-3-on
[1H]-NMR (CDCl₃): 8,68breit 1H, 7,3 bis 6,8 zus. 4H, 3,12 d 1H, 1,97 heptett 1H, 1,09 d 6H.
2-n-Propyl-2H-1,4-benzothiazin-3-on

### E) 2-Phenyl-2-H-3-chlor-1,4-benzoxazin

Zu 0,22 g 2-Phenyl-1,4-benzoxazin-3-on in 3 ml Acetonitril werden 0,1 ml Phosphoroxychlorid und 1,2 mmol Triethylamin gegeben. Nach 12 Stunden rühren bei Raumtemperatur wird eingeengt. Das Rohprodukt kann für weitere Umsetzungen verwendet werden.

Auf die gleiche Weise werden hergestellt:
2-Methyl-3-chlor-1,4-benzoxazin
2,6-Dimethyl-3-chlor-1,4-benzoxazin
2-Methyl-3,6-dichlor-1,4-benzoxazin

### F) 2-Ethyl-1,4-benzoxazin-3-thion

0,25 g 2-Ethyl-1,4-benzoxazin-3-on werden in 2 ml Pyridin mit 95 mg Phosphorpentasulfid gerührt. Die Reaktion wird durch Kochen auf Rückfluss vervollständigt. Es wird auf Wasser gegossen, extrahiert mit Ethylacetat und die organische Phase mit Sole gewaschen. Man trocknet mit Magnesiumsulfat und engt ein. Nach Säulenchromatographie mit Hexan / Ethylacetat resultieren 220 mg Produkt.
[1H]-NMR (CDCI₃): 7,09 bis 6,8 m 4H, 4,39 dd 1 H, 2,1 bis 1,8 m 4H, 1,11 tr 3H.

Auf die gleiche Weise werden hergestellt:
2,2-Dimethyl-1,4-benzoxazin-3-thion
Die Ausbeute beträgt 76%
[1H]-NMR (CDCl₃): 9,8 breit 1H, 7,10 bis 6,84 m 4H, 1,69 s 6H.
2-(2-Phenylethyl)-2H-1,4-benzoxazin-3-thion
[1H]-NMR (CDCl₃): 9,7 1H, 7,33 bis 6,95 m 8H, 6,87 dd 1H, 4,91 dd 1H, 2,9 m 2H, 2,4 und 2,2 m je 1 H.
Schmelzpunkt 134 °C.
Ausbeute 78%.
2-Phenyl-2H-1,4-benzoxazin-3-thion
[1 H]-NMR (CDCl₃): 9,9 1H, 7,5 m 2H, 7,3 m 3H, 7,1 bis 6,8 m 4H, 6,1 s 1 H.
Schmelzpunkt 191 °C.
Ausbeute 90%
2-Benzyl-1,4-benzoxazin-3-thion
2-Propyl-1,4-benzothiazin-3-thion
2-Propyl-1,4-benzoxazin-3-thion
2-Ethyl-1,4-benzothiazin-3-thion
2-(2-Propyl)-1,4-benzothiazin-3-thion
2-(2-Propyl)-1,4-benzoxazin-3-thion
2-tert. Butyl-1,4-benzoxazin-3-thion
2,5-Dimethyl-1,4-benzoxazin-3-thion
2,7-Dimethyl-1,4-benzoxazin-3-thion
2,8-Dimethyl-1,4-benzoxazin-3-thion
2,6-Dimethyl-1,4-benzoxazin-3-thion
2-n-Butyl-1,4-benzothiazin-3-thion
2-n-Butyl-1,4-benzoxazin-3-thion
2-n-Hexyl-1,4-benzoxazin-3-thion
2-Carboxymethyl-1,4-benzoxazin-3-thion
2,2-spiro-Butyl-1,4-benzoxazin-3-thion
2-Acetyl-1,4-benzoxazin-3-thion
2-Methyl-6-chlor-1,4-benzoxazin-3-thion
2-Methyl-7-methylcarboxy-1,4-benzoxazin-3-thion
2-Methyl-6,8-dichlor-1,4-benzoxazin-3-thion
2-Methyl-6-tert-butyl-1,4-benzoxazin-3-thion
2,6,7-Trimethyl-1,4-benzoxazin-3-thion
2.6,8-Trimethyl-1,4-benzoxazin-3-thion

### G) 2-(2-Hydroxyethyl)-1,4-benzoxazin-3-on

2.18 g 2-Aminophenol und 1,1 Equivalente 2-Brom-γ-butyrolacton werden in 25 ml DMF mit 4 g Kaliumcarbonat versetzt. Nach 6 Stunden erwärmen auf 75 °C wird auf Wasser gegossen, mit Essigester extrahiert, die organische Phase mit Natriumsulfat getrocknet und eingeengt. Es resultieren 40% Ausbeute.
[1H]-NMR (DMSO): 10,6 breit 1H, 6,9 m 4H, 4,65 dd 1H, 4,52 breit 1H, 3,6 m 2H, 1,9 m 2H.

Daraus erhält man in oben beschriebenen Verfahren unter Anwendung einer Schutzgruppe:
2-(2-Hydroxyethyl)-1,4-benzoxazin-3-thion

### H) 2-(2-Bromoethyl)-1,4-benzoxazin-3-on

580 mg 2-(2-Hydroxyethyl)-1,4-benzoxazin-3-on werden mit je 1 Equivalent Triphenylphosphin und Tetrabrommethan in 50 ml Dichlormethan gerührt. Nach 12 Stunden bei RT ist die Reaktion beendet. Man engt die Mischung ein und gibt alles auf eine Säule. Nach Säulenchromatografie erhält man die Titelverbindung in einer Ausbeute von 79%
[1H]-NMR (CDCl₃): 8,35 breit 1 H, 7,05 bis 6,8 m 4H, 4,77 dd 1 H, 3,67 m 2H, 2,5 m 2H.

### I) 2-(2-Methylthio-ethyl)-1,4-benzoxazin-3-on

586 mg 2-(2-Bromoethyl)-1,4-benzoxazin-3-on werden in einer Methanol-DMF Mischung mit 245 mg Natriumthiomethylat unter Rühren versetzt. Nach 4 Stunden wird eingeengt . Nach Säulenchromatographie erhält man die die Titelverbindung in einer Ausbeute von 53%.

Daraus erhält man in oben beschriebenen Verfahren:
2-(2-Methylthio-ethyl)-1,4-benzoxazin-3-thion

### Beispiel 1

### 2-Methyl-3-amino-1,4-benzoxazin

2 g 2-Aminophenol werden in Methanol mit 1 Equivalent Natriummethylat deprotoniert und die Lösung bei Eisbadtemperatur tropfenweise mit 3 ml D,L-2-Chlor-2-methylacetonitril versetzt. Nach Ablauf der Alkylierungsreaktion wird unter Zugabe von katalytischen Mengen Natriummethylat erwärmt. Man gießt auf Wasser, extrahiert mit Essigester, die organische Phase wird mit Magnesiumsulfat getrocknet und eingeengt. Es resultieren 1,3 g Rohprodukt, das umkristallisiert wird.
[1H]-NMR (CDCl₃): 7,30 dd 1H, 6,94 dd 2H, 6,86 dd 1H, 5,05 breit 2H, 4,62 q 7Hz 1 H, 1,49 d 7Hz 3H.
Schmelzpunkt 153°C.
Die gleiche Verbindung erhält man analog zu Beispiel 3 aus dem Thion.

### Beispiel 2

### 2-Phenyl-2-H-3-amino-1,4-benzoxazin

1 mmol 2-Phenyl-2H-3-chlor-1,4-benzoxazin in 2 ml Acetonitril werden bei 8 bar Ammoniakdruck im Autoklaven mehrere Stunden gerührt. Es wird eingeengt. Das Rohprodukt wird mit Wasser gewaschen und mit Magnesiumsulfat getrocknet. Danach wird mittels Säulenchromatografie in Essigester neben 2-Phenyl-2H-4H-1,4-benzoxazin-3-on eine Fraktion von 45% Produkt erhalten.
Schmelzpunkt 232 °C Zers.

Auf die gleiche Weise werden hergestellt:
2,6-Dimethyl-3-amino-1,4-benzoxazin
2-Methyl-3-amino-6-chlor-1,4-benzoxazin
[1H]-NMR (CDCl₃):7,01 d 1 H, 6,90 dd 8/1 Hz 1 H, 6,79 d 1 H, 4,61 q 7Hz, 1,49 d 3H.
Die Ausbeute beträgt 46%

### Beispiel 3

### 2-Ethyl-3-amino-1,4-benzoxazin

Die Verbindung wird durch Rühren von 210 mg 2-Ethyl-1,4-benzoxazin-3-thion in 15 ml ges. Ammoniaklösung in Methanol nach 2 Tagen bei Raumtemperatur erhalten. Das Rohprodukt wird nach Einengen durch Säulenchromatografie gereinigt. Es resultieren 144 mg Produkt.
[1H]-NMR (CDCl₃): 7,05 bis 6,82 m 4H, 4,41 dd 1 H, 1,9 bis 1,6 m 4H, 1,07 tr 3H.

Auf die gleiche Weise werden hergestellt:
2,2-Dimethyl-3-amino-1,4-benzoxazin
[1H]-NMR (CDCl₃): 7,03 bis 6,81 m 4H, 1,50 s 6H.
Schmelzpunkt 202 °C.
66% Ausbeute.
2,7-Dimethyl-3-amino-1,4-benzoxazin
2,8-Dimethyl-3-amino-1,4-benzoxazin
2, 5-Dimethyl-3-amino-1,4-benzoxazin
2,6-Dimethyl-3-amino-1,4-benzoxazin
2-(2-Phenylethyl)-2H-3-amino-1,4-benzoxazin
Farbloses Öl.Die Ausbeute beträgt 83%.
2-Methyl-3-amino-7-carboxymethyl-1,4-benzoxazin
[1H]-NMR (DMSO): 7,49dd, 7,30d, 7,1 s breit, 6,90 d je 1H, 4,72 q 1 H, 3,8 s 3H, 1,29 d 3H.
2-Methyl-3-amino-6,8-dichlor-1,4-benzoxazin
2-Benzyl-3-amino-1,4-benzoxazin
2-Propyl-3-amino-1,4-benzothiazin
2-Propyl-3-amino-1,4-benzoxazin
[1H]-NMR (CDCl₃): 7,1 bis 6,8 m 4H, 4,50 dd 1H, 1,9 bis 1,5 m 4H, 0,99 tr 3H.
2-(2-Propyl)-3-amino-1,4-benzothiazin
[1H]-NMR (DMSO): 6,7 breit, 7,2 bis 6,75 zus. 4H, 3,02 d 1H, 1,51 heptett 1 H, 0,91 dd 6H.
2-(2-Propyl)-3-amino-1,4-benzoxazin
[1H]-NMR (DMSO): 6,7 breit, 6,75 m 4H, 4,22 d 1H, 1,90 heptett 1H, 0,99 d 3H, 0,88 d 3H.
2-n-Butyl-3-amino-1.4-benzothiazin
2-n-Butyl-3-amino-1,4-benzoxazin
2-*tert*.-Butyl-3-amino-1,4-benzoxazin
2-n-Butyl-3-amino-1,4-benzoxazin
2-Ethyl-3-amino-1,4-benzothiazin
2-Methyl-3-amino-6-*tert*-butyl-1,4-benzoxazin
Schmelzpunkt: 125-130° C
2,6,7-Trimethyl-3-amino-1,4-benzoxazin
halberstarrtes Öl
2,6,8-Trimethyl-3-amino-1,4-benzoxazin
2-n-Hexyl-3-amino-1,4-benzoxazin
2-Carboxymethyl-3-amino-1,4-benzoxazin
2,2-spiro-Butyl-3-amino-1,4-benzoxazin
[1 H]-NMR (MeOH): 6,9 bis 6,7 m 4H, 2,45, 2,20,1,84 je m 2H.
2-(2-Hydroxyethyl)-3-amino-1,4-benzoxazin.
2-(2-Methylthio-ethyl)-3-amino-1,4-benzoxazin,
[1H]-NMR (CDCl₃): 7,05 bis 6,83 m 4H, 4,77 dd 1 H, 2,70 m 2H, 2,11 s 3H, 2 bis 1,8 m 2H

### Beispiel 4

### 2-(S)-Methyl-3-amino-6-chlor-1,4-benzoxazin und 2-(R)-Methyl-3-amino-6-chlor-1,4-benzoxazin

Einige Milligramm 2-(R,S)-Methyl-3-amino-6-chlor-1,4-benzoxazin werden auf einer chiralen HPLC-Säule (Chirapak AD, 250x4,6) mit Hexan / Isopropanol / Ethanol bei 1 ml / Minute eluiert und bei 220 nm detektiert. Man fängt zwei Fraktionen auf.

### Beispiel 5

### 2-Methyl-3-amino-6-chlor-1,4-benzoxazin-Hydrochlorid

Zu 0,051 g 2-Methyl-3-amino-6-chlor-1,4-benzoxazin in THF werden bei Eisbadtemperatur 1,0 ml etherische 1 n Salzsäure gegeben. Man rührt nach und läßt einige Zeit stehen. Die Kristalle werden durch dekantieren abgetrennt. Die Ausbeute beträgt 67%. [1H]-NMR (DMSO): 10,1 s breit 1H, 7,36 d 1H, 7,19dd 1H, 7,09 d 1H, 5,33 q 1H, 1,51 d 3H.

## Patentansprüche

1. Verbindungen der Formel I, deren tautomere und isomere Formen und Salze 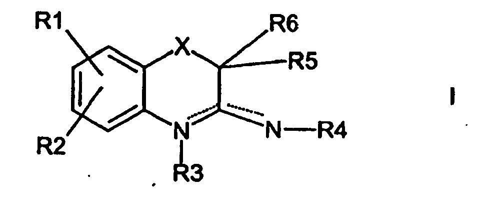 worin
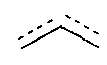 eine Doppelbindung,
X -O- oder -S(O)ₘ-,
R¹ und R² unabhängig voneinander Wasserstoff, Halogen, S(O)ₙ-R⁷, OR⁷, COOR⁷, NR⁷R⁸, C(=NR⁷)-NHR⁸, C₁₋₆-Alkyl, C₂₋₆-Alkenyl, C₂₋₆-Alkynyl oder-S-C(=NR⁷)-NHR⁸,
R³ und R⁴ unabhängig voneinander Wasserstoff, C₁₋₁₂-Alkyl, Phenyl, CO-NR⁹R¹⁰, CSNR⁹R¹⁰, COR⁹, CSR⁹, COOR⁹, OH, O-C₁₋₆-Alkyl,
und
R⁵ Halogen, C₁₋₈-Alkoxy,S(O)ₚ-C₁₋₆-Alkyl,C₁₋₈-Alkylcarbonyl,C₁₋₈-Alkyl,-C₃₋₁₀₋Cycloalkyl, Phenyl oder einen C₁₋₈-Alkylrest, der mit Phenyl, Halogen, Hydroxy, S(O)ₙR¹¹, NO₂, OR¹¹, COOR¹¹, NR¹¹R¹², Cyano, -C(=NR¹¹)-NHR¹² oder-S-C(=NR¹¹)-NHR¹² substituiert ist.
R⁶ Wasserstoff oder C₁₋₃-Alkyl, das gegebenenfalls gemeinsam mit R⁵ einen 3-, 4-oder 5-gliedrigen Spirocyclus bildet,
R⁷, R⁸ und R¹¹, R¹² gleich oder verschieden Wasserstoff, C₁₋₆-Alkyl, Phenyl oder C₃₋₇-Cycloalkyl,
R9 und R¹⁰ unabhängig voneinander Wasserstoff, Phenyl, Benzyl, C₃₋₇-Cycloalkyl oder C₁₋₆-Alkyl,
m, n, p 0, 1 oder 2 ist.

2. Verbindungen gemäß Anspruch 1, worin R³ und R⁴ Wasserstoff bedeuten.

3. Verbindungen gemäß Anspruch 1 oder 2, worin R⁶ Wasserstoff ist.

4. Verbindungen gemäß den Ansprüchen 1 - 3, worin R⁵ C₁₋₈-Alkyl ist.

5. 2,6-Dimethyl-3-amino-1,4-benzoxazin
2,5-Dimethyl-3-amino-1,4-benzoxazin
2,7-Dimethyl-3-amino-1,4-benzoxazin
2,8-Dimethyl-3-amino-1,4-benzoxazin
2-Methyl-2H-3-amino-1,4-benzoxazin
2-Methyl-2H-3-amino-1,4-benzothiazin
2-Ethyl-3-amino-1,4-benzothiazin
2-Ethyl-3-amino-1,4-benzoxazin
2-n-Propyl-3-amino-1,4-benzoxazin
2-Phenyl-2-H-3-amino-1 ,4-benzoxazin
2-(2-Hydroxyethyl)-3-amino-1,4-benzoxazin
2-(2-Methylthio-ethyl)-3-amino-1,4-benzoxazin
nach Anspruch 1.

6. Arzneimittel enthaltend eine Verbindung nach einem der vorhergehenden Ansprüche

7. Verwendung der Verbindungen der Formel I nach einem der Ausprüche 1-5 zur Herstellung eines Arzneimittels zur Behandlung von Erkrankungen, die durch Stickstoffmonoxid Synthase ausgelöst werden.

8. Verfahren zur Herstellung der Verbindungen der Formel I nach einem der Ansprüche 1-5 **dadurch**, daß man
a) eine Verbindung der Formel IIa oder IIb oder deren Salze 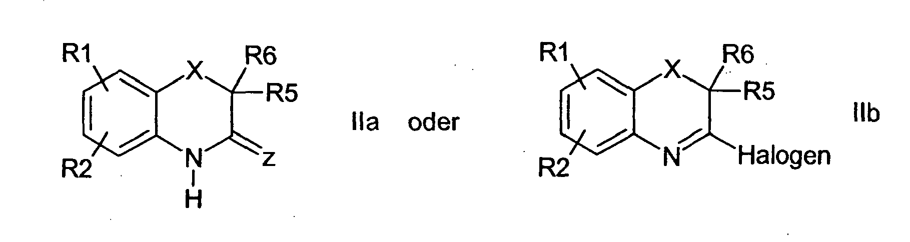 worin
R¹. R² und X die obige Bedeutung haben und Z Sauerstoff oder Schwefel ist, mit Ammoniak oder primären oder sekundären Aminen umsetzt oder
b) eine Verbindung der Formel III 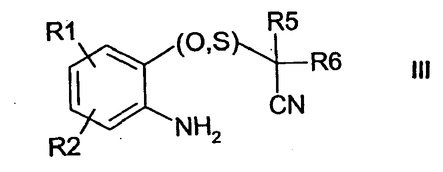 worin
R¹, R², R⁵, R⁶ die obige Bedeutung haben, cyclisiert und gewünschtenfalls anschließend Sulfide oxidiert, Ester verseift, Säuren verestert, Hydroxygruppen verethert oder acyliert, Amine acyliert, alkyliert, diazotiert, halogeniert. NO₂ einführt oder reduziert, mit Thiohamstoffderivaten umsetzt, die Isomeren trennt oder die Salze bildet.

9. Verbindung gemäß einem der Ansprüche 1-5 der Herapeutischen Verwendung.

## Claims

1. Compounds of formula I, tautomeric and isomeric forms and salts thereof, 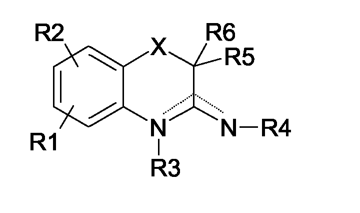 wherein
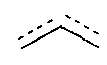 is a double bond,
X is -O- or -S(O)ₘ-,
R¹ and R² are each independently hydrogen, halogen, S(O)ₙ-R⁷, OR⁷, COOR⁷, NR⁷R⁸, C(=NR⁷)-NHR⁸, C₁₋₆alkyl, C₂₋₆ alkenyl, C₂₋₆ alkynyl, or -S-C(=NR⁷)-NHR⁸,
R³ and R⁴ are each independently hydrogen, C₁₋₁₂ alkyl, phenyl, CO-NR⁹R¹⁰, CSNR⁹R¹⁰, COR⁹, COOR⁹, OH, O-C₁₋₆ alkyl,
and
R⁵ is halogen, C₁₋₈ alkoxy, S(O)ₚ-C₁₋₆ alkyl, C₁₋₈ alkylcarbonyl, C₁₋₈ alkyl, C₃₋₁₀ cycloalkyl, phenyl or a C₁₋₈ alkyl group substituted with phenyl, halogen, hydroxy, S(O)ₙR¹¹, NO₂, OR¹¹, NR¹¹R¹², cyano, -C(=NR¹¹)NHR¹² or -S-C(=NR¹¹)-NHR¹²,
R6 is hydrogen or C₁₋₃ alkyl optionally forming together with R⁵ a 3-, 4- or 5-membered spiro cycle,
R⁷, R⁸ and R¹¹, R¹² are the same or different hydrogen, C₁₋₆ alkyl, phenyl or C₃₋₇ cycloalkyl,
R⁹ and R¹⁰ are each independently hydrogen, phenyl, benzyl, C₃₋₇ cycloalkyl or C₁₋₆ alkyl,
m, n, p are 0, 1 or 2.

2. Compounds according to claim 1, wherein R³ and R⁴ are hydrogen.

3. Compounds according to claims 1 or 2, wherein R⁶ is hydrogen.

4. Compounds according to claims 1-3, wherein R⁵ is C₁₋₈ alkyl.

5. 2,6-dimethyl-3-amino-1,4-benzoxazin,
2,5-dimethyl-3-amino-1,4-benzoxazin,
2,7-dimethyl-3-amino-1,4-benzoxazin,
2,8-dimethyl-3-amino-1,4-benzoxazin,
2-methyl-2H-3-amino-1,4-benzoxazin,
2-methyl-2H-3-amino-1,4-benzothiazin,
2-ethyl-3-amino-1,4-benzothiazin,
2-ethyl-3-amino-1,4-benzoxazin,
2-n-propyl-3-amino-1,4-benzoxazin,
2-phenyl-2H-3-amino-1,4-benzoxazin,
2-(2-hydroxyethyl)-3-amino-1,4-benzoxazin,
2-(2-bromoethyl)-3 -amino-1,4-benzoxazin,
2-(2-methylthio-ethyl)-3-amino-1,4-benzoxazin,
according to claim 1.

6. Medicament comprising a compound according to any one of the preceding claims.

7. Use of the compounds of formula I according to any one of claims 1 to 5 for the preparation of a medicament for the treatment of disease-states caused by nitric oxide synthase.

8. Process for the preparation of compounds of formula I according to any one of claims 1 to 5, **characterized in that**
a) a compound of formula IIa or IIb or salts thereof 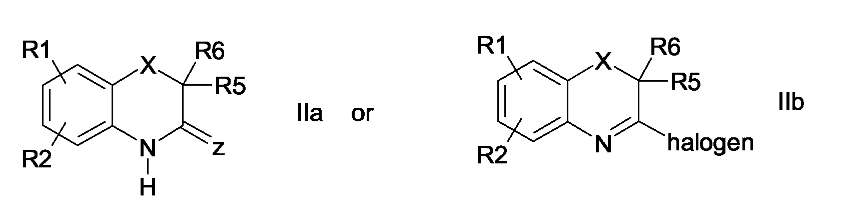 wherein
R¹, R² and X have the meaning as defined above and Z is oxygen or sulfur, is reacted with ammonia or primary or secondary amines, or
b) a compound of formula III 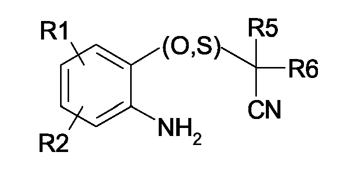 wherein
R¹, R², R⁵, R⁶ have the meaning as defined above, is cyclized and - if desired - subsequently sulfides are oxidized, esters are saponified, acids are esterified, hydroxy groups are acylated or etherified, amines are acylated, alkylated, diazotized, halogenated, NO₂ is introduced or reduced, reacted with thiourea derivatives, isomers are separated or salts are formed.
9. Compound according to any one of claims 1 to 5 for therapeutic use.

## Revendications

1. Composés de formule I, leurs tautomères et leurs formes isomères et sels 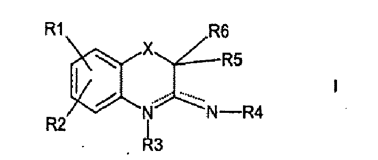 dans laquelle
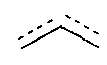 représente une double liaison,
X représente un groupe -O- ou un groupe -S(O)ₘ-,
R¹ et R² représentent, indépendamment l'un de l'autre, un atome d'hydrogène, un atome d'halogène, un groupe S(O)ₙ-R⁷, un groupe OR⁷, un groupe COOR⁷, un groupe NR⁷R⁸, un groupe C(=NR⁷)-NHR⁸, un groupe alkyle en C₁₋₆, un groupe alcényle en C₂₋₆, un groupe alcynyle en C₂₋₆ ou un groupe -S-C(=NR⁷)-NHR⁸,
R³ et R⁴ représentent, indépendamment l'un de l'autre, un atome d'hydrogène, un groupe alkyle en C₁₋₁₂, un groupe phényle, un groupe CO-NR⁹R¹⁰, un groupe CSNR⁹R¹⁰, un groupe COR⁹, un groupe CSR⁹, un groupe COOR⁹, un groupe OH, un groupe O-C₁₋₆-alkyle,
et
R⁵ représente un atome d'halogène, un groupe alcoxy en C₁₋₈, un groupe S(O)ₚ₋C₁₋₆-alkyle, un groupe C₁₋₈-alkylcarbonyle, un groupe alkyle en C₁₋₈, un groupe cycloalkyle en C₃₋₁₀, un groupe phényle ou un radical alkyle en C₁₋₈, qui est substitué par un groupe phényle, un atome d'halogène, un groupe hydroxy, un groupe S(O)ₙR¹¹, un groupe NO₂, un groupe OR¹¹, un groupe COOR¹¹, un groupe NR¹¹R¹², un groupe cyano, un groupe -C(=NR¹¹)-NHR¹² ou un groupe -S-C(=NR¹¹)-NHR¹²,
R⁶ représente un atome d'hydrogène ou un groupe alkyle en C₁₋₃, qui forme éventuellement, conjointement avec R⁵, un cycle spiro à 3, 4 ou 5 chaînons,
R⁷, R⁸ et R¹¹, R¹², identiques ou différents, représentent un atome d'hydrogène, un groupe alkyle en C₁₋₆, un groupe phényle ou un groupe cycloalkyle en C_{3-7,}
R⁹ et R¹⁰ représentent, indépendamment l'un de l'autre, un atome d'hydrogène, un groupe phényle, un groupe benzyle, un groupe cycloalkyle en C₃₋₇ ou un groupe alkyle en C_{1-6,}
m, n, p valent 0, 1 ou 2.

2. Composés selon la revendication 1, dans lesquels R³ et R⁴ représentent un atome d'hydrogène.

3. Composés selon la revendication 1 ou 2, dans lesquels R⁶ représente un atome d'hydrogène.

4. Composés selon les revendications 1 à 3, dans lesquels R⁵ représente un groupe alkyle en C₁₋₈.

5. 2,6-Diméthyl-3-amino-1,4-benzoxazine,
2,5-diméthyl-3-amino-1,4-benzoxazine,
2,7-diméthyl-3-amino-1,4-benzoxazine,
2,8-diméthyl-3-amino-1,4-benzoxazine,
2-méthyl-2H-3-amino-1,4-benzoxazine,
2-méthyl-2H-3-amino-1,4-benzothiazine,
2-éthyl-3-amino-1,4-benzothiazine,
2-éthyl-3-amino-1,4-benzoxazine,
2-n-propyl-3-amino-1,4-benzoxazine,
2-phényl-2-H-3-amino-1,4-benzoxazine,
2-(2-hydroxyéthyl)-3-amino-1,4 benzoxazine,
2-(2-bromoéthyl)-3-amino-1,4-benzoxazine,
2-(2-méthylthioéthyl)-3-amino-1,4-benzoxazine,
selon la revendication 1.

6. Médicament comprenant un composé selon l'une quelconque des revendications précédentes.

7. Utilisation des composés de formule I selon les revendications 1 à 5 pour la préparation d'un médicament destiné au traitement de maladies qui sont provoquées par le monoxyde d'azote synthase.

8. Procédé de préparation des composés de formule I selon l'une quelconque des revendications 1 à 5, **caractérisé en ce que**
a) un composé de formule IIa ou IIb ou leurs sels 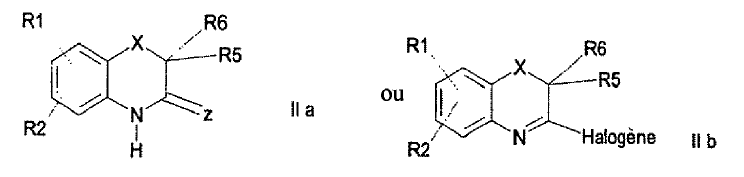 R¹, R² et X présentent la signification ci-dessus et Z représente un atome d'oxygène ou un atome de soufre, sont mis à réagir avec de l'ammoniac ou des amines primaires ou secondaires, ou
b) un composé de formule III 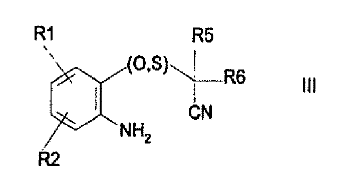 dans laquelle
R¹, R², R5, R⁶ présentent la signification ci-dessus, est cyclisé et ensuite, si on le souhaite, les sulfures sont oxydés, les esters sont hydrolysés, les acides sont estérifiés, les groupes hydroxy sont éthérifiés ou acylés, les amines sont acylées, alkylées, diazotées, halogénées, des groupes NO₂ sont introduits ou réduits, on met à réagir avec des dérivés de thio-urée, on sépare les isomères ou on forme les sels.
9. Composé selon l'une quelconque des revendications 1 à 5 pour une utilisation thérapeutique.
